# EUROPEAN PATENT SPECIFICATION

(11) **EP 2 781 226 B1**
(45) Date of publication and mention of the grant of the patent: **12.08.2015**
(21) Application number: 14160707.7
(22) Date of filing: 19.03.2014
(51) Int. Cl.: A61L 2/22, A61L 2/18

(54) **Helmet sanitizing/disinfecting device**
Helmreinigungsvorrichtung
Apparail de nettoyage de casque

(30) Priority: 20.03.2013 IT MI20130423
(43) Date of publication of application: 24.09.2014
(73) Proprietor: Allegrini S.p.A., 24050 Grassobbio (BG) (IT)
(72) Inventor: DE BIASI, Giuseppe, 24050 Grassobbio (Berganmo) (IT)
(74) Representative: Sgobba, Marco

(56) References cited:
- WO-A1-2012/131740
- FR-A1- 2 791 585
- JP-A- H10 192 798
- JP-A- 2003 311 225
- US-B1- 6 558 620

## Description

### TECHNICAL FIELD

The present invention refers to a helmet disinfecting device. The present invention is particularly useful in disinfecting motorcycle helmets, though such use is only one example of possible application.

### BACKGROUND OF THE INVENTION

Motorcyclists in particular, and however all users of protection helmets, are particularly keen on the care of their helmets. In fact, a motorcyclist always ensures that his/her helmet is thoroughly polished, as regards the cap (so as to outline the livery) as well as regards the visor (for obvious safety purposes). In order to meet such needs, many products for cleaning the helmet are commercially available, which are capable of restoring the shininess and clarity of the livery of the helmet such as to make the latter practically new. In addition, specific products for cleaning, or better for cleansing and disinfecting, the internal of the helmet, i.e. the part of the helmet which is at contact with the head of the user when the helmet is worn. Usually the operations of disinfecting the internal of the helmet imply a predetermined non-use period of the same, for example given the time required to allow the internal of the helmet to dry after being wet or moistened. Thus, it is not always possible to have a helmet whose internal was perfectly disinfected, especially if the user forgets to follow the cleaning operations once back home.

In addition, in case of long trips or during summer, the internal of the helmet, being at contact with the skin of the user, is impregnated with sweat and thus remains until one is back to the place where the cleaning products are.

In addition, in shops in which helmets are sold, the latter are worn by the potential purchasers to test the comfort and functionality thereof, besides appreciating the aesthetic value thereof. It may thus occur that a client of a shop may be wearing a helmet which has already been worn several times, with the entailed poor hygiene conditions.

An example of helmet cleaner is described in document JPH10192798A where a cleaning tank has means for fixing the helmet inside, a helmet cleaning means and drying means the helmet is cleaned and then dried. another helmet cleaner is described in document JP2003311225A.

The Applicant observed that it could be advantageous to be able to disinfect the internal of the helmet every time the helmet is removed, and whenever the helmet is not worn.

In addition, the Applicant observed that it would be extremely advantageous to be able to disinfect the helmet whenever the motorcyclist is forced to stop, for example for motorcycle maintenance purposes or breaks or refuelling.

In this context, the technical task of the present invention is to propose a helmet disinfecting device capable of disinfecting and cleansing the internal of helmets on demand, i.e. whenever required.

In particular, an aim of the present invention is to provide a helmet disinfecting device capable of disinfecting the internal of a helmet automatically and anywhere.

### SUMMARY OF THE INVENTION

According to the present invention, the technical task and the aims of the present invention are attained through a helmet disinfecting device according to the features of one or more of the attached claims.

### BRIEF DESCRIPTION OF THE DRAWINGS

The features and advantages of the present invention shall be evident from the following detailed description of an embodiment of a helmet disinfecting device according to the present invention, provided by way of non-limiting example with reference to the attached drawings, wherein:
- figure 1 shows a schematic perspective view of a helmet disinfecting device according to the present invention,
- figure 2 shows a schematic front view of the device of figure 1,
- figure 3 represents a functional scheme of some parts of the distributor of figure 1.

### DETAILED DESCRIPTION OF THE INVENTION

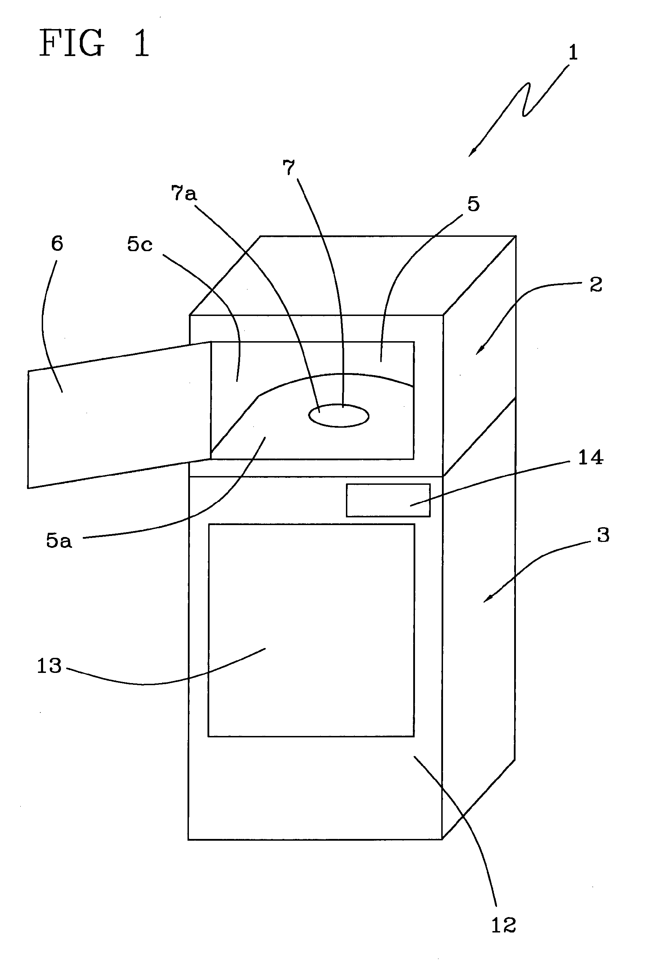

A helmet disinfecting device is overall indicated with reference number 1 in figure 1. The device 1 comprises two areas at least functionally separate from each other, and in particular a first area 2 and a second area 3. The first area 2 is provided to receive a helmet to be disinfected and comprises at least one dispenser nozzle 4 capable of dispensing a disinfectant aerosol. In the context of the present invention the term disinfectant aerosol means the dispersion of a disinfectant liquid in a gas, for example air, wherein the particles of the disinfectant liquid have dimensions preferably comprised between 0.5 and 60 microns. The disinfectant liquid is a liquid capable of at least partly eliminating bacterial loads, algae, moulds and foul odours. The disinfectant liquid may be of the type made and used only under the authorisation of the Ministry of Health (medical device) or of free production and sale.

The first area 2 comprises a compartment 5 for housing a helmet, in which the helmet may be placed for disinfecting operations. The dimensions of the housing compartment 5 are such to allow an easy introduction of full helmets. The housing compartment 5 comprises a base surface 5a configured for receiving a helmet, a top surface 5b opposite to the base surface 5a and preferably a sidewall 5c substantially curved and without corners which develops between the base surface and the top surface. In an embodiment, the sidewall 5c, in a plan view, comprises two substantially straight segments, opposite with respect to each other and connected by an arc. In an embodiment, the housing compartment 5 is made of stainless steel to facilitate the cleaning and disinfecting operations thereof.

The housing compartment 5 is closed by a port 6 preferably arranged at the front area of the housing compartment 5.

The dispenser nozzle 4 is arranged in the housing compartment 5, advantageously at the top surface thereof. In the preferred embodiment of the invention, the dispenser nozzle 4 is oriented in the housing compartment 5 so as to dispense the disinfectant aerosol downwards, i.e. towards the base surface 5a of the housing compartment 5. In embodiments of the invention not illustrated, the dispenser nozzles may be more than one, each one of which is arranged at the top surface 5b of the housing compartment 5. Preferably, the pressure with which the aerosol exits from the dispenser nozzle 4 is slightly greater than the atmospheric pressure, for example it is comprised, in absolute value, between 1.05 and 1.2 atm.

Within the housing compartment 5, and in particular in proximity of the base surface 5a, there are also arranged support members 7 for the helmet configured so as to firmly hold the helmet during the activation of the dispenser nozzle 4. In particular, the support members 7 hold the helmet so as to expose the internal part thereof (i.e. the part intended to contact the head of the user) upwards. Thus, the inner part of the helmet is directly exposed to the action of the dispenser nozzle 4. In other words, the dispenser nozzle 4 and the support members 7 cooperate to allow the disinfectant aerosol to reach and impact the inner part of the helmet in a uniform manner. In the embodiment illustrated in the attached figures, the support members 7 are formed by a circular hole 7a arranged at the base surface of the housing compartment 5 and adapted to constrain an annular gasket (not illustrated) configured to engage the external cap of an inverted helmet. The helmet is fitted on such annular gasket to be received into the housing compartment 5. In an alternative embodiment not shown in the attached figures, the first area 2 comprises alternative support members and possibly removable configured to support the helmet to be disinfected.

Preferably, the first area 2 comprises suctioning members 8 adapted to evacuate from the housing compartment 5 the disinfectant aerosol dispensed by the dispenser nozzle 4. Such suctioning members 8 may be formed by a fan adapted to evacuate the aerosol from the housing compartment 5 to emit it into the environment outside the helmet disinfecting device 1.

Preferably, the first area 2 further comprises a filter (not illustrated) cooperating with the suctioning members 8 to filter the aerosol to be evacuated before it is liberated into the external environment.

Preferably, the first area 2 comprises ventilation members 9 arranged inside the housing compartment 5 and adapted to facilitate the uniform distribution of the disinfectant aerosol dispensed by the dispenser nozzle 4.

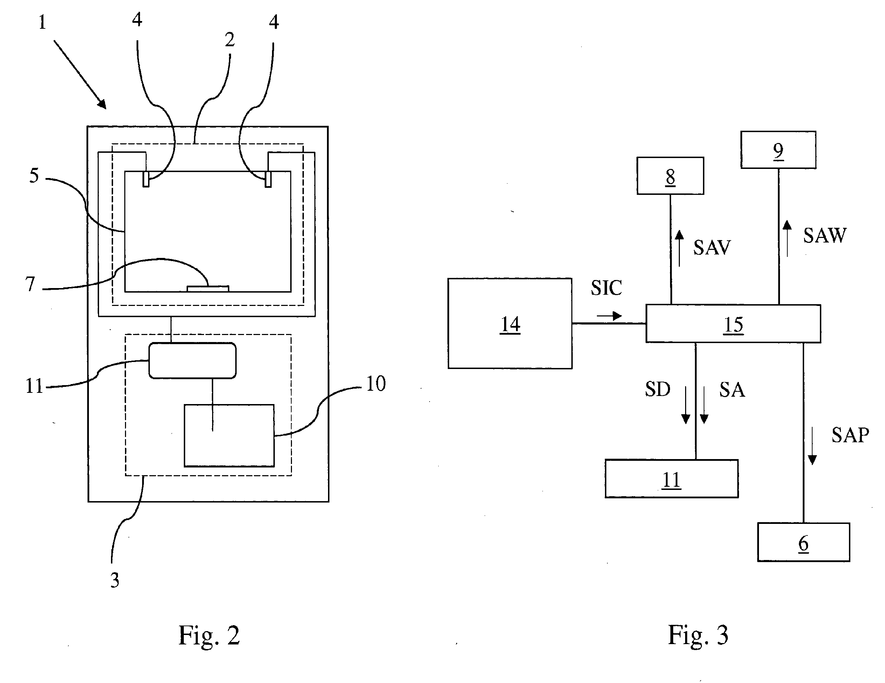

The second area 3 is provided to contain the disinfectant liquid and to let the disinfectant aerosol flow to the first area 2 and in particular to the dispenser nozzle 4. Preferably, the disinfectant liquid is diluted with water at 50% in volume. The second area 3 (schematised in figure 2) comprises a tank 10 for the disinfectant liquid and an aerosol generator device 11 active on the tank 10 for generating a disinfectant aerosol. The aerosol generator device 11 is in fluid communication with the dispenser nozzle 4, for dispensing the disinfectant aerosol in the housing compartment 5. In the preferred embodiment of the invention, the aerosol generator device 11 is, preferably, a ultrasonic atomiser. The formation of the aerosol in the ultrasonic atomiser occurs through the contact of the disinfectant liquid with the surface of a piezoelectric crystal which vibrates at frequencies between 20 kHz and 10 MHz, preferably 1.7 MHz. The ultrasonic atomiser is capable of transforming the disinfectant liquid into drops with average diameter ranging from 0.5 to 60 microns, preferably from 1 to 10 microns, even more preferably from 3 to 6 microns. The aerosol generator device 11 generates an aerosol which has a density Da greater than density of air in the same pressure and temperature conditions, and in particular at ambient pressure and temperature. Therefore, the aerosol dispensed by the dispenser nozzle 4 at the top surface 5b of the housing compartment 5, due to gravity, tends to move downwards reaching the base surface 5a of the housing compartment. This allows an optimal diffusion and distribution of the disinfectant aerosol in the housing compartment 5.

In an embodiment, the tank 10 for the disinfectant liquid comprises a level sensor configured to mark the reaching of the minimum level of the disinfectant liquid, for example by lighting a LED light and/or by emitting a sound and/or the signal on a display. Preferably, such signalling systems are arranged in a position which can be seen by a user so as to facilitate the prompt refuelling of the tank 10 for the disinfectant liquid.

In the preferred embodiment of the invention, the second area 3 is a box-shaped body 12 defining an internal cavity containing the tank 10 for the disinfectant liquid and the aerosol generator device 11. Such internal cavity has an opening engaged by one port 13 adapted to allow access to the cavity. In the embodiment shown in the attached figures, the first area 2 is arranged above the second area 3 so as to obtain a single containment structure. An embodiment not shown in the attached figures, provides that the first area 2 is arranged laterally adjacent to the second area 3. In a further embodiment, the helmet disinfecting device 1 comprises a plurality of first areas 2, wherein each first area 2 is provided to receive a respective helmet to be disinfected.

The aerosol generator device 11 is of the electrical type. For such purpose, in the second area 3 transformers are arranged, which are connectable to the electrical power supply systems which supply all electrical devices of the helmet disinfecting device 1, or sources of electrical power (such as for example solar panels, accumulators, batteries and the like) can be provided, directly housed in the second area 3.

The helmet disinfecting device 1 further comprises a payment interface 14 provided to activate a disinfecting cycle following payment by a user. Such payment interface may be of the token, prepaid card, banknotes, credit card type or a combination of the above. The payment interface is configured to send a start of the disinfecting cycle signal SIC to a command and control unit 15 following payment by a user. In an alternative embodiment, the payment interface 14 may be replaced or combined with a different actuation system, for example of the button type or through a touch-screen display or other systems which allow starting and stopping a disinfecting cycle.

Preferably, the command and control unit 15, schematised in figure 3, is a PLC and it is provided to manage disinfection cycles. The command and control unit 15 is provided to send activation SA and de-activation SD signals to the aerosol generator device 11, in order to allow the dispenser nozzle 4 to dispense disinfectant aerosol and to prevent it from dispensing disinfectant aerosol. The activation signal SA is generated by the command and control unit 15 when a signal representing the start of the cycle SIC is received. Such signal SIC is generated by the payment interface 14 or by an activation device such as for example a button. The command and control unit 15 is also provided to generate and send to the port 6 an opening signal SAP so that the port can be opened and so that it is possible to access into the internal of the housing compartment 5.

In use, a user introduces the helmet into the first area 2, and in particular into the housing compartment 5. Then, the port 6 is closed. In the preferred embodiment of the invention, the user must pay for the service through the payment interface 14. Upon paying, the payment interface 14 generates the cycle start signal SIC. In alternative embodiments, the closure of the port 6 or a dedicated button generate the cycle start signal SIC. Upon receiving the cycle start signal SIC, the activation signal SA generated and sent by the command and control unit 15 activates the dispensing of the nozzle 4 for a predefined time ranging from 10 to 90 seconds, preferably of about 60 seconds. In any case, the activation time of the aerosol generator device 11 is such to allow a complete saturation of the housing compartment 5 with disinfectant aerosol. In addition, it should be observed that the disinfectant liquid, when dispensed in form of aerosol, does not wet, at least macroscopically, the helmet, thus allowing an immediate use of the same at the end of the disinfecting cycle. In other words, the exposure of the helmet to the disinfectant aerosol does not require that the helmet is subjected to drying operations in that the disinfectant aerosol is not capable of significantly wetting the helmet (both due to the period of stay of the same in the housing compartment and the dispensed amount). Once the predefined time of activation elapses, the command and control unit 15 sends the de-activation signal SD which stops the dispensing of the disinfectant aerosol. The disinfectant aerosol totally, or at least partly, eliminates bacteria from the part of the helmet at contact with the head of the user. In an embodiment, the de-activation signal SD may be sent following the opening of the port 6 during the disinfecting cycle through a sensor present on the port 6 or by pressing a cycle stop button.
Preferably, the command and control unit 15 generates and sends an activation signal SAW to the ventilation members 9. The ventilation members 9 remain active over the entire duration of dispensing the disinfectant aerosol by the dispenser nozzle 4. Such ventilation members 9 force the circulation of the disinfectant aerosol in the housing compartment 5. This operation allows an optimal distribution of the disinfectant aerosol in the housing compartment 5. Subsequently, once the time comprised between 30 and 90 seconds from the emission of the de-activation signal SD of the aerosol generator device 11 elapses, the command and control unit 15 is configured to send an activation signal SAV to the suctioning members 8, to activate the same for a period comprised between 5 and 30 seconds. At this point, the command and control unit 15 generates the opening signal SAP and sends it to the port 6, which opens and allows to collect the helmet perfectly disinfected. It should be observed that the opening signal SAP of the port 6 is sent by the command and control unit 15 after a period of at least 30 seconds from the activation of the suctioning members 8. In an embodiment, following the opening of the port 6 during the performance of a disinfecting cycle, the command and control unit 15 is configured to send the activation signal SAV to the suctioning members 8 to suction the disinfectant aerosol from the housing compartment 5. The port 6 is adapted to allow a user to visually monitor the inner part of the housing compartment 5. Regarding this, the first area 2 comprises a plurality of LED lights adapted to light the inner part of the housing compartment 5 with at least two different colours. The two colours comprise a first colour indicating the activation of a disinfecting cycle and a second colour indicating the end of the disinfecting cycle.

It should be observed that the amount of disinfectant liquid consumed by the aerosol generator device 5 during a disinfecting cycle is comprised between 10 and 40 millilitres.

The invention attains the preset objects, given that the disinfecting device allows to disinfect a helmet automatically, rapidly, efficiently and without wetting or moistening the helmet. The disinfecting device may for example be installed in mechanics' workshops, shops, stop bays and service bays and anywhere a motorcyclist may stop. Thus, when the motorcyclist stops, for example to rest, the helmet may be disinfected. In addition, when the disinfecting device is placed in a shop, the device may be used for disinfecting the helmets which are worn by the potential clients, so that each helmet on sale is always in perfect hygiene conditions.

Obviously, a person skilled in the art may be able to perform many modifications and variants to the aforedescribed configurations with the aim of meeting the contingent and specific requirements. For example, the two areas 2, 3 may be physically separated from each other, the tank 10 of disinfectant liquid may be arranged at a distant position with respect to the device, or the number of dispenser nozzles 4 may vary. Such variants and modifications fall within the scope of protection of the invention as defined by the claims that follow.

## Claims

1. Helmet disinfecting device (1) comprising a first area (2) arranged to receive a helmet to be disinfected and comprising a compartment (5) for housing said helmet, said housing compartment (5) comprising a base surface (5a) configured for receiving said helmet, **and comprising a circular hole (7a) adapted to constrain an annular gasket, said annular gasket being configured to engage a capsized** helmet, and a top surface (5b) opposite to said base surface (5a), said housing compartment (5) further comprising a front opening engaged by a port (6), adapted to allow the insertion and the extraction of said helmet from said housing compartment (5), said first area (2) further comprising at least one dispenser nozzle (4) positioned at said top surface (5b), provided for dispensing a disinfectant aerosol within said housing compartment (5), said helmet disinfecting device (1) further comprising a second area (3), different from the first one (2), comprising a tank (10) for disinfectant liquid and an aerosol generator device (11) active on said tank (10) for the disinfectant liquid, in fluid communication with said at least one dispenser nozzle (4), for generating a disinfectant aerosol, said helmet disinfecting device (1) further comprising a command and control unit (15) configured to send at least activation (SA) and de-activation (SD) signals to said aerosol generator device (11).

2. Helmet disinfecting device (1) according to claim 1, wherein said housing compartment (5) comprises a curved sidewall (5c) which develops between said base surface (5a) and said top surface (5b).

3. Helmet disinfecting device (1) according to claim 1 or 2, wherein said aerosol generator device (11) is an ultrasonic atomiser.

4. Helmet disinfecting device (1) according to any one of the preceding claims, wherein said first area (2) and said second area (3) are arranged adjacent or superimposed with respect to each other and they are obtained in a single containment structure.

5. Helmet disinfecting device (1) according to any one of the preceding claims, wherein said first area (2) comprises suctioning members (8) adapted to evacuate said disinfectant aerosol dispensed by said at least one dispenser nozzle (4) from said housing compartment (5).

6. Helmet disinfecting device (1) according to claim **5**, wherein said first area (2) comprises a filter cooperating with said suctioning members (8) to filter said disinfectant aerosol to be evacuated.

7. Helmet disinfecting device (1) according to any one of the preceding claims, wherein said first area (2) comprises ventilation members (9) arranged within said housing compartment (5) and adapted to force the circulation of said disinfectant aerosol dispensed by said at least one dispenser nozzle (4).

8. Helmet disinfecting device (1) according to any one of the preceding claims, comprising a payment interface (14) configured to be active on said command and control unit (15) upon a payment by a user.

9. Helmet disinfecting device (1) according to claim 9, wherein said payment interface (14) comprises interfaces of the token and/or prepaid card and/or banknotes and/or credit card type.

10. Helmet disinfecting device (1) according to any one of the preceding claims, comprising a plurality of first areas (2,2',2ⁿ), wherein each first area is arranged to receive a helmet to be disinfected.

11. Helmet disinfecting device (1) according to any one of the preceding claims, wherein said command and control unit (15) is configured to receive a signal representing the start of the cycle (SIC) and generating said activation signal (SA) upon receiving the cycle start signal (SIC); said activation signal (SA) activating said aerosol generator device (11) for a predefined period comprised between 10 and 90 seconds.

12. Helmet disinfecting device (1) according to any one of the preceding claims, wherein said first area (2) comprises a plurality of LED lights adapted to light the inner part of said housing compartment (5) with at least two different colours, wherein a first colour indicates the activation of a disinfecting cycle and a second colour indicates the end of said disinfecting cycle.

13. Helmet disinfecting device (1) according to any one of the preceding claims, wherein said aerosol generator device (11) is configured for consuming an amount of said disinfectant liquid comprised between 10 and 40 millilitres during a disinfecting cycle.

## Patentansprüche

1. Helmdesinfektionsvorrichtung (1), die einen ersten Bereich (2) umfasst, der eingerichtet ist, um einen zu desinfizierenden Helm aufzunehmen und ein Fach (5) zum Aufnehmen des Helms umfasst, wobei dieses Aufnahmefach (5) eine Grundfläche (5a), die zum Aufnehmen des Helms gestaltet ist und ein kreisrundes Loch (7a) umfasst, das zum Halten einer ringförmigen Dichtung eingerichtet ist, wobei diese ringförmige Dichtung gestaltet ist, um mit einem umgekehrten Helm in Eingriff zu sein, und eine der Grundfläche (5a) entgegengesetzte obere Fläche (5b) umfasst, wobei dieses Aufnahmefach (5) ferner eine mit einer Tür (6) in Eingriff befindliche vorderseitige Öffnung umfasst, die geeignet ist, um das Einführen und das Herausnehmen des Helms aus dem Aufnahmefach (5) zu ermöglichen, wobei der erste Bereich (2) ferner mindestens eine auf der oberen Fläche (5b) angeordnete Abgabedüse (4) umfasst, die zum Abgeben eines Desinfektionsmittel-Aerosols im Aufnahmefach (5) vorgesehen ist, wobei diese Helmdesinfektionsvorrichtung (1) ferner einen vom ersten (2) verschiedenen zweiten Bereich (3) umfasst, der einen Tank (10) für eine Desinfektionsmittelflüssigkeit und eine auf dem Tank (10) für die Desinfektionsmittelflüssigkeit arbeitende, mit der mindestens einen Abgabedüse (4) in Fluidverbindung stehende Aerosolerzeugungsvorrichtung (11) zum Erzeugen eines Desinfektionsmittel-Aerosols umfasst, wobei die Helmdesinfektionsvorrichtung (1) ferner eine Steuer- und Kontrolleinheit (15) umfasst, die gestaltet ist, um mindestens Aktivierungs- (SA) und Deaktivierungssignale (SD) an die Aerosolerzeugungsvorrichtung (11) zu senden.

2. Helmdesinfektionsvorrichtung (1) nach Anspruch 1, wobei das Aufnahmefach (5) eine gebogene Seitenwand (5c) umfasst, die zwischen der Grundfläche (5 a) und der oberen Fläche (5b) ausgebildet ist.

3. Helmdesinfektionsvorrichtung (1) nach Anspruch 1 oder 2, wobei die Aerosolerzeugungsvorrichtung (11) ein Ultraschallzerstäuber ist.

4. Helmdesinfektionsvorrichtung (1) nach einem der vorhergehenden Ansprüche, wobei der erste Bereich (2) und der zweite Bereich (3) nebeneinanderliegend oder übereinanderliegend angeordnet und in einer einzigen Umschließungskonstruktion ausgebildet sind.

5. Helmdesinfektionsvorrichtung (1) nach einem der vorhergehenden Ansprüche, wobei der erste Bereich (2) Saugelemente (8) umfasst, die geeignet sind, das von der mindestens einen Abgabedüse (4) abgegebene Desinfektionsmittel-Aerosol aus dem Aufnahmefach (5) auszutragen.

6. Helmdesinfektionsvorrichtung (1) nach Anspruch 5, wobei der erste Bereich (2) ein mit den Saugelementen (8) zusammenarbeitendes Filter umfasst, um das auszutragende Desinfektionsmittel-Aerosol zu filtern.

7. Helmdesinfektionsvorrichtung (1) nach einem der vorhergehenden Ansprüche, wobei der erste Bereich (2) Ventilationselemente (9) umfasst, die in dem Aufnahmefach (5) angeordnet und geeignet sind, die Zirkulation des von der mindestens einen Abgabedüse (4) abgegebenen Desinfektionsmittel-Aerosols zu erzwingen.

8. Helmdesinfektionsvorrichtung (1) nach einem der vorhergehenden Ansprüche, die eine Bezahlschnittstelle (14) umfasst, die gestaltet ist, um auf eine Bezahlung eines Benutzers hin auf die Steuer- und Kontrolleinheit (15) einzuwirken.

9. Helmdesinfektionsvorrichtung (1) nach Anspruch 9, wobei die Bezahlschnittstelle (14) Schnittstellen vom Gutschein- und/oder Guthabenkarten- und/oder Banknoten- und/oder Kreditkartentyp umfasst.

10. Helmdesinfektionsvorrichtung (1) nach einem der vorhergehenden Ansprüche, die eine Vielzahl von ersten Bereichen (2, 2', 2ⁿ) umfasst, wobei jeder erste Bereich eingerichtet ist, um einen zu desinfizierenden Helm aufzunehmen.

11. Helmdesinfektionsvorrichtung (1) nach einem der vorhergehenden Ansprüche, wobei die Steuer- und Kontrolleinheit (15) gestaltet ist, um ein Signal zu empfangen, das den Start des Zyklus (SIC) repräsentiert, und das Aktivierungssignal (SA) nach Empfang des Zyklusstartsignals (SIC) zu erzeugen; wobei dieses Aktivierungssignal (SA) die Aerosolerzeugungsvorrichtung (11) für einen vorbestimmten Zeitraum, vorzugsweise zwischen 10 und 90 Sekunden, aktiviert.

12. Helmdesinfektionsvorrichtung (1) nach einem der vorhergehenden Ansprüche, wobei der erste Bereich (2) eine Vielzahl von LED-Leuchten umfasst, die geeignet sind, um das Innere des Aufnahmefachs (5) mit mindestens zwei verschiedenen Farben zu beleuchten, wobei eine erste Farbe die Aktivierung eines Desinfektionszyklus anzeigt und eine zweite Farbe das Ende des Desinfektionszyklus anzeigt.

13. Helmdesinfektionsvorrichtung (1) nach einem der vorhergehenden Ansprüche, wobei die Aerosolerzeugungsvorrichtung (11) gestaltet ist, um während eines Desinfektionszyklus eine Menge der Desinfektionsmittelflüssigkeit zwischen 10 und 40 Millilitern zu verbrauchen.

## Revendications

1. Dispositif de désinfection de casque (1) comprenant une première zone (2) agencée pour recevoir un casque à désinfecter et comprenant un compartiment (5) pour loger ledit casque, ledit compartiment de logement (5) comprenant une surface de base (5a) configurée pour recevoir ledit casque, et comprenant un trou circulaire (7a) adapté pour bloquer un joint annulaire, ledit joint annulaire étant configuré pour engager un casque renversé, et une surface supérieure (5b) opposée à ladite surface de base (5a), ledit compartiment de logement (5) comprenant en outre une ouverture avant engagée par un port (6), adaptée pour permettre l'introduction et l'extraction dudit casque dudit compartiment de logement (5), ladite première zone (2) comprenant en outre au moins une buse de distribution (4) positionnée au niveau de ladite surface supérieure (5b), prévue pour distribuer un aérosol de désinfectant à l'intérieur dudit compartiment de logement (5), ledit dispositif de désinfection de casque (1) comprenant en outre une deuxième zone (3), différente de la première zone (2), comprenant un réservoir (10) pour le liquide désinfectant et un dispositif générateur d'aérosol (11) actif sur ledit réservoir (10) pour le liquide désinfectant, en communication de fluide avec ladite au moins une buse de distribution (4), pour générer un aérosol de désinfectant, ledit dispositif de désinfection de casque (1) comprenant en outre une unité de commande et de contrôle (15) configurée pour envoyer au moins des signaux d'activation (SA) et de désactivation (SD) audit dispositif générateur d'aérosol (11).

2. Dispositif de désinfection de casque (1) selon la revendication 1, dans lequel ledit compartiment de logement (5) comprend une paroi latérale courbe (5c) qui se développe entre ladite surface de base (5a) et ladite surface supérieure (5b).

3. Dispositif de désinfection de casque (1) selon la revendication 1 ou 2, dans lequel ledit dispositif générateur d'aérosol (11) est un pulvérisateur à ultrasons.

4. Dispositif de désinfection de casque (1) selon l'une quelconque des revendications précédentes, dans lequel ladite première zone (2) et ladite deuxième zone (3) sont agencées adjacentes ou superposées l'une par rapport à l'autre et elles sont obtenues dans une unique structure de confinement.

5. Dispositif de désinfection de casque (1) selon l'une quelconque des revendications précédentes, dans lequel ladite première zone (2) comprend des éléments aspirants (8) adaptés pour évacuer dudit compartiment de logement (5) ledit aérosol de désinfectant distribué par ladite au moins une buse de distribution (4).

6. Dispositif de désinfection de casque (1) selon la revendication 5, dans lequel ladite première zone (2) comprend un filtre coopérant avec lesdits éléments aspirants (8) pour filtrer ledit aérosol de désinfectant à évacuer.

7. Dispositif de désinfection de casque (1) selon l'une quelconque des revendications précédentes, dans lequel ladite première zone (2) comprend des éléments de ventilation (9) agencés à l'intérieur dudit compartiment de logement (5) et adaptés pour forcer la circulation dudit aérosol de désinfectant distribué par ladite au moins une buse de distribution (4).

8. Dispositif de désinfection de casque (1) selon l'une quelconque des revendications précédentes, comprenant une interface de paiement (14) configurée pour être active sur ladite unité de commande et de contrôle (15) lors d'un paiement par un utilisateur.

9. Dispositif de désinfection de casque (1) selon la revendication 9, dans lequel ladite interface de paiement (14) comprend des interfaces du type jeton et/ou carte prépayée et/ou billets de banque et/ou carte de crédit.

10. Dispositif de désinfection de casque (1) selon l'une quelconque des revendications précédentes, comprenant une pluralité de premières zones (2, 2', 2"), dans lequel chaque première zone est agencée pour recevoir un casque à désinfecter.

11. Dispositif de désinfection de casque (1) selon l'une quelconque des revendications précédentes, dans lequel ladite unité de commande et de contrôle (15) est configurée pour recevoir un signal représentant le début du cycle (SIC) et générant ledit signal d'activation (SA) à la réception du signal de début de cycle (SIC) ; ledit signal d'activation (SA) activant ledit dispositif générateur d'aérosol (11) pendant une période prédéfinie comprise entre 10 et 90 secondes.

12. Dispositif de désinfection de casque (1) selon l'une quelconque des revendications précédentes, dans lequel ladite première zone (2) comprend une pluralité de lampes à LED adaptées pour éclairer la partie interne dudit compartiment de logement (5) avec au moins deux couleurs différentes, dans lequel une première couleur indique l'activation d'un cycle de désinfection et une deuxième couleur indique la fin dudit cycle de désinfection.

13. Dispositif de désinfection de casque (1) selon l'une quelconque des revendications précédentes, dans lequel ledit dispositif générateur d'aérosol (11) est configuré pour consommer une quantité dudit liquide désinfectant comprise entre 10 et 40 millilitres durant un cycle de désinfection.
